# EUROPÄISCHE PATENTSCHRIFT

(11) **EP 1 114 621 B1**
(45) Veröffentlichungstag und Bekanntmachung des Hinweises auf die Patenterteilung: **19.11.2008**
(21) Anmeldenummer: 00204146.5
(22) Anmeldetag: 23.11.2000
(51) Int. Cl.: A61B 19/00

(54) **Anordnung zur Darstellung von Schichtbildern**
Apparatus for displaying images
Dispositif pour representer d'images

(30) Priorität: 02.12.1999 DE 19958407
(43) Veröffentlichungstag der Anmeldung: 11.07.2001
(73) Patentinhaber: Philips Intellectual Property & Standards GmbH, 20099 Hamburg (DE); Koninklijke Philips Electronics N.V., 5621 BA Eindhoven (NL)
(72) Erfinder: Grass, Michael, Dr., 52064 Aachen (DE); Koehler, Thomas, Dr., 52064 Aachen (DE)
(74) Vertreter: Volmer, Georg

(56) Entgegenhaltungen:
- WO-A-99/00052
- US-A- 5 836 869
- US-A- 5 911 036
- US-A- 5 954 648

## Beschreibung

Die Erfindung betrifft eine Anordnung zur Darstellung von Schichtbildern während der Behandlung eines Untersuchungsobjekts sowie ein medizinisches Instrument zur teilweisen Einführung in ein Untersuchungsobjekt während einer Behandlung.

Eine Anordnung und ein Verfahren der eingangs genannten Art sind aus der US 5,954,648 bekannt. Dort wird mittels eines optischen Positionsmeßsystems die Position eines medizinischen Behandlungsinstruments, z.B. einer Biopsienadel, bestimmt und in präoperativ mittels einer Röntgeneinrichtung erstellte Röntgenschichtbilder eingeblendet. Dazu sind an dem medizinischen Instrument LEDs angebracht, deren räumliche Position mittels einer Kamera des Positionsmeßsystems bestimmt werden kann. Durch ein geeignetes Registrierungsverfahren können diese räumlichen Positionen in Positionen relativ zu der Abbildungsgeometrie der Röntgeneinrichtung und damit relativ zu den Röntgenschichtbildern umgerechnet werden.

Bei einer solchen Anordnung, die insbesondere bei der bildgeführten Chirurgie Einsatz findet, werden dem behandelnden Arzt ein oder mehrere Schichtbilder mit eingeblendeter aktueller Position des medizinischen Instruments gezeigt, um ihm die Führung des Instruments innerhalb des Untersuchungsobjekts zu erleichtern. Die Orientierung und Lage der dargestellten Bilder wird dabei eingangs festgelegt, z.B. wird immer ein Schichtbild dargestellt, das in einer Ebene liegt, auf dem das medizinische Instrument senkrecht steht und das durch die Spitze des medizinischen Instruments verläuft. Die Lage des dargestellten Schichtbildes wird dann beispielsweise mit dem medizinischen Instrument mitgeführt oder durch Eingabe an einem Steuerrechner verändert. Wünschenswert wäre jedoch, wenn die Lage und Orientierung der dargestellten Schichtbilder schnell, einfach und insbesondere interaktiv durch den behandelnden Arzt selbst möglich wäre, so dass er sich immer einen Überblick über die Anatomie des Untersuchungsobjekts selbst verschaffen kann.
Der Erfindung liegt deshalb die Aufgabe zugrunde, eine Anordnung der eingangs genannten Art so auszugestalten, dass eine solche einfache, schnelle und interaktive Veränderung der Lage und Orientierung der dargestellten Schichtbilder möglich ist. Außerdem sollen ein entsprechendes medizinisches Instrument und ein entsprechendes Verfahren angegeben werden.

Diese die Anordnung betreffende Aufgabe wird durch eine Anordnung des Anspruch 1 gelöst.

Die Erfindung geht dabei von der Erkenntnis aus, dass geeignete Einstellmittel zur Veränderung der Orientierung der dargestellten Schichtbilder dort anzuordnen sind, wo der behandelnde Arzt deren Einstellung einfach und interaktiv verändern kann. Dies ist bei einer Behandlung mittels eines medizinischen Instruments dieses medizinische Instrument selbst. Durch eine Veränderung der Einstellung dieser Einstellmittel kann somit unmittelbar und möglichst ohne Zeitverzögerung eine anders orientierte Schicht dargestellt werden. Beispielsweise kann der Winkel zwischen einer durch die Spitze des medizinischen Instruments verlaufenden Schicht und der Längsachse des medizinischen Instruments verändert werden. Oder es eine Schicht, die durch die Längsachse des medizinischen Instruments verläuft, um das medizinische Instrument herum rotiert werden. Die dargestellten Schichtbilder selbst können entweder aus einem dreidimensionalen präoperativ erstellten Bilddatensatz, der mittels einer geeigneten bildgebenden Einrichtung wie einer Röntgeneinrichtung, einer Computertomographieeinrichtung, einem Kernspintomographen oder einer Ultraschalleinrichtung akquiriert wurde, oder intraoperativ während der Behandlung mittels einer solchen bildgebenden Einrichtung erstellt werden.

Bei einer Weiterbildung der Erfindung sind die Einstellmittel mit einer Steuereinheit gekoppelt, beispielsweise über eine Draht- oder Funkverbindung, um an die Steuereinheit die aktuelle Einstellung der Einstellmittel zu übertragen und dadurch die Darstellung und ggf. vorherige Erstellung der Schichtbilder zu beeinflussen.

In einer alternativen Weiterbildung der Erfindung sind die Einstellmittel derart ausgestaltet, dass deren Einstellung mittels der Meßmittel bestimmbar ist. Beispielsweise können bei Verwendung eines bekannten optischen Positionsmeßsystems, was in einer weiteren Ausgestaltung der Erfindung vorgesehen ist, an den Einstellmitteln wie an dem medizinischen Instrument selbst LEDs vorgesehen sein, deren räumliche Positionen bestimmt werden können, woraus sich die Einstellung der Einstellmittel ermitteln läßt. Bevorzugt arbeitet ein solches Positionsmeßsystem mit Infrarot-LEDs und Infrarotkameras, denkbar sind jedoch auch andere Möglichkeiten wie elektromagnetische Sender und Empfänger.

Die Ausgestaltungen der Erfindung gemäß den Ansprüchen 4 bis 6 sollen eine möglichst einfache und leichte Bedienung der Einstellmittel ermöglichen. Bevorzugt soll anhand der Einstellmittel auch ein die Orientierung eines dargestellten Schichtbildes kennzeichnender Wert, z.B. der Winkel zwischen einer dargestellten Schicht und einer Bezugsschicht, ablesbar sein.

Besonders einfache und vorteilhafte Ausgestaltungen der Einstellmittel sind in den Ansprüchen 7 bis 9 angegeben. Derartige Einstellmittel sind einfach herzustellen, lassen sich leicht bedienen und können bei Bedarf an vorhandene medizinische Instrumente einfach angebracht und wieder entfernt werden.

In einer bevorzugten Ausgestaltung werden Schichtbilder dargestellt, die durch die Längsachse des medizinischen Instruments verlaufen. Mittels der Einstellmittel können diese Schichtbilder um diese Längsachse herum rotiert werden.

Bei der besonders bevorzugten Ausgestaltung gemäß Anspruch 13, bei der eine Röntgeneinrichtung vorgesehen ist, können sowohl Schichtbilder dargestellt werden, die aus einem präoperativ erfaßten Bilddatensatz erstellt werden als auch intraoperativ erstellte Röntgenschicht- oder Röntgenprojektionsbilder. Die Einstellmittel können dabei so ausgestaltet sein, dass sowohl die Orientierung der intraoperativ erstellten Röntgenbilder als auch der aus dem präoperativen Bilddatensatz erstellten Schichtbilder beeinflußt werden kann. Durch die Einstellmittel kann somit auch die Erstellung von Röntgenbildern mittels der Röntgeneinrichtung, die beispielsweise eine C-Bogen-Röntgeneinrichtung sein kann, gesteuert werden.

Eine derartige Ausgestaltung weiterbildend ist gemäß Anspruch 14 vorgesehen, dass die Einstellmittel in den Röntgenbildern abbildbar ausgestaltet sind und dass die Meßmittel derart ausgestaltet sind, dass aus den Abbildungen der Einstellmittel deren Einstellung bestimmbar ist. Bei einer solchen Weiterbildung kann auf ein weiter oben beschriebenes separates Positionsmeßsystem verzichtet werden. Sowohl die Position des medizinischen Instruments relativ zu dem präoperativ erfaßten Bilddatensatz als auch die aktuelle Einstellung der Einstellmittel werden dabei dadurch ermittelt, dass wenigstens ein Teil des medizinischen Instruments und die Einstellmittel in intraoperativ erfaßten Röntgenbildern, z.B. Röntgenprojektionsaufnahmen, abgebildet werden. Aus diesen Röntgenbildern, die beispielsweise während einer Behandlung in festen Zeitabständen erstellt werden, wird mittels eines geeigneten Registrierungsverfahren die Position des medizinischen Instruments bestimmt und dabei die Einstellung der Einstellmittel "abgelesen". Bevorzugt sind die Einstellmittel dazu derart ausgestaltet und werden die Röntgenbilder derart erstellt, dass die Bestimmung der Einstellung eindeutig und auf einfache Weise möglich ist, so dass dies auch rechnergestützt erfolgen kann.

Die Erfindung umfaßt auch ein medizinisches Instrument gemäß Anspruch 15 welches in geeigneter Weise zur Anwendung bei einer erfindungsgemäßen Anordnung ausgestaltet sind. Insbesondere weist das medizinische Instrument geeignete Einstellmittel auf, wobei die Einstellmittel wie oben beschrieben ausgestaltet sein können.

Die Erfindung wird nachfolgend anhand der Zeichnungen näher erläutert. Es zeigen:
- Fig. 1: eine erfindungsgemäße Anordnung mit einer Röntgeneinrichtung und einem Positionsmeßsystem und
- Fig. 2a, 2b: ein erfindungsgemäßes medizinisches Instrument in zwei unterschiedlichen Orientierungen.

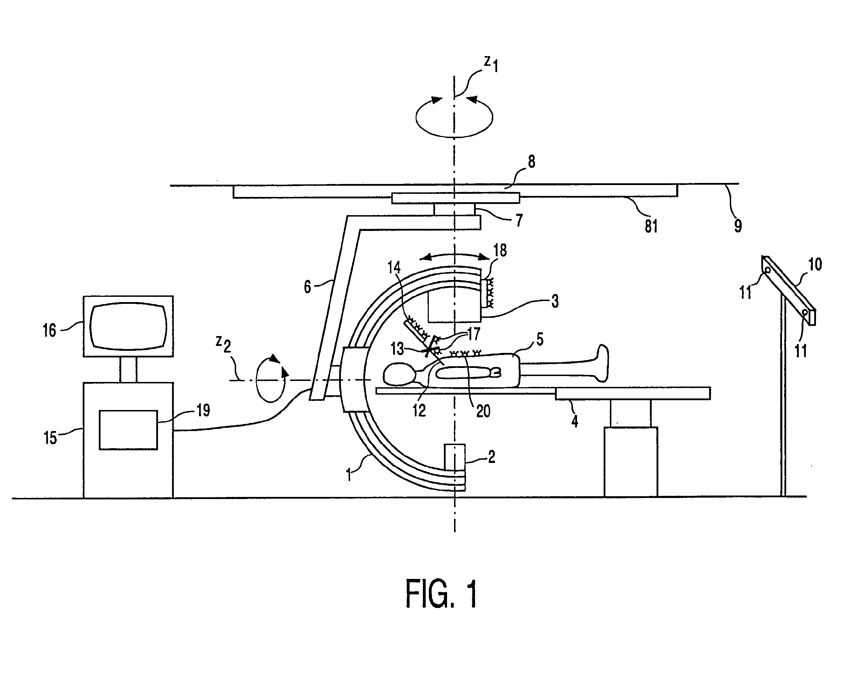

Die in Fig. 1 gezeigte erfindungsgemäße Anordnung weist eine Röntgeneinrichtung auf mit einem C-Bogen 1, an dessen erstem Ende eine Röntgenröhre 2 und an dessen anderem Ende ein Röntgendetektor 3, z.B. ein Bildverstärker, angeordnet sind. Mit dieser Röntgeneinrichtung können Röntgenprojektionsaufnahmen von einem auf einem Tisch 4 liegenden Patienten 5 aus unterschiedlichen Röntgenpositionen erstellt werden, wozu der C-Bogen 1 die drei räumlichen Achsen um z₁, z₂ und (nicht gezeigt) z₃ rotierbar ausgestaltet ist. Der C-Bogen 1 ist über eine Haltevorrichtung 6, ein Drehgelenk 7 und einen Schlitten 8, der in einem Schienensystem 81 in horizontaler Richtung verfahrbar ist, an der Decke 9 befestigt.

Weiter ist ein Positionsmeßsystem der bekannten Art mit einer Kameraeinheit 10 mit zwei Infrarotkameras 11 vorgesehen. Mittels dieser Kameraeinheit 10 kann die Position von im Untersuchungsbereich angeordneten Infrarot-Leuchtdioden im Raum ermittelt werden. An einem medizinischen Instrument 12, das während der Behandlung des Patienten 5 in diesen teilweise eingeführt wird, sind drei solche Leuchtdioden 14 angeordnet, um die Position des medizinischen Instruments 12 im Raum zu messen und in eine Position des medizinischen Instruments 12 relativ zu einem präoperativ mit der gezeigten Röntgeneinrichtung erfaßten dreidimensionalen Bilddatensatz des Behandlungsbereichs des Patienten 5 umzurechnen. Weiter sind an dem erfindungsgemäß ausgestalteten medizinischen Instrument 12 Einstellmittel 13 angeordnet, auf deren Aufbau und Funktionsweise weiter unten näher eingegangen wird. Diese Einstellmittel 13 weisen ebenfalls Leuchtdioden 17 auf, um die aktuelle Einstellung der Einstellmittel 13 mit dem Positionsmeßsystem zu bestimmen. Um auch die Position der bildgebenden Einheit der Röntgeneinrichtung und die Position des Patienten zu bestimmen, sind sowohl an dem Röntgendetektor 3 weitere drei Leuchtdioden 13 als auch am Patienten 5 weitere Leuchtdioden 20 angeordnet.

Die Röntgeneinrichtung und das Positionsmeßsystem werden von einer Steuereinheit 15 gesteuert. An diese werden auch die ermittelten Daten (Röntgenbilder, Positionsdaten) übermittelt, wo sie in einer Recheneinheit 19 verarbeitet werden. Um den Arzt während der Behandlung zu unterstützen, können auf einem Monitor 16 verschiedene Bilder dargestellt werden.

Mit der gezeigten Röntgeneinrichtung können sowohl vor einer Behandlung eine Reihe von Röntgenprojektionsbildern aus unterschiedlichen Röntgenpositionen erstellt werden, aus denen ein dreidimensionaler Bilddatensatz, dreidimensionale Rekonstruktionsbilder und Röntgenschichtbilder erstellt werden kann, als auch während der Behandlung Röntgenprojektionsbilder erstellt werden. Weil dabei jeweils auch die Position der bildgebenden Einheit bestimmt wird, läßt sich die Lage von intraoperativ ermittelten Röntgenprojektionsbildern relativ zu einem präoperativ ermittelten Bilddatensatz bestimmen. Auch die relative Position des medizinischen Instruments 12 relativ zu dem präoperativ ermittelten Bilddatensatz läßt sich so bestimmen. Die aktuelle Position des medizinischen Instruments 12 läßt sich also in Schichtbilder einblenden, die aus dem präoperativ ermittelten Bilddatensatz erstellt und während der Behandlung auf dem Monitor 16 angezeigt werden.

Um diese Funktionalität zu ermöglichen, ist es meist erforderlich, dass bei der Erstellung des präoperativen Bilddatensatzes an dem Patienten spezielle Marker angebracht sind, die in den Bildern mit abgebildet werden und dass die Marker auch bei der Behandlung noch an den selben Stellen angebracht sind. Mittels eines speziellen Pointers, dessen Position im Raum mit dem Positionsmeßsystem bestimmt werden kann, werden dann diese Marker einzeln angefahren, um deren Positionen im Raum zu bestimmen. Dadurch wird die Lage des präoperativen Bilddatensatzes im Raumkoordinatensystem oder im Patientenkoordinatensystem bestimmt.

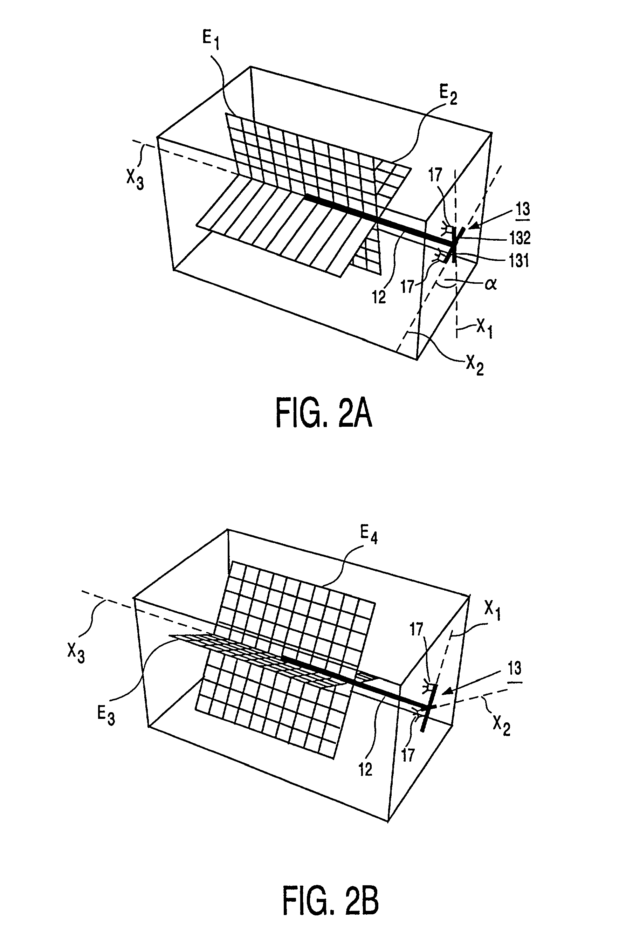

Die Orientierung solcher Schichtbilder läßt sich mit Hilfe der Einstellmittel 13 auf einfache Weise manuell und interaktiv während der Behandlung des Patienten 5 durch den Arzt verändern, was anhand von Fig. 2a und 2b näher erläutert werden soll. Dort ist ein medizinisches Instrument 12, z.B. eine stark vereinfachte Biopsienadel, gezeigt, an deren Ende die Einstellmittel 13 angeordnet sind. Diese umfassen zwei senkrecht zueinander und senkrecht zur Längsachse x₃ des medizinischen Instruments 12 angeordnete kurze Stäbe 131 und 132. An jedem dieser Stäbe 131, 132 ist eine Infrarot-Leuchtdiode 17 angeordnet, deren Position mit dem Positionsmeßsystem bestimmbar ist. Die Stäbe 131 und 132 sind so ausgestaltet, dass sie in der durch ihre beiden Längsachsen x₁, x₂ definierten Ebene um die Längsachse x₃ des medizinischen Instruments 12 herum rotierbar sind, wobei jedoch der Winkel α zwischen den beiden Stäben 131, 132 fest ist.

Während einer Behandlung werden dem Arzt auf dem Monitor zwei Röntgenschichtbilder dargestellt, die in den beiden gezeigten Ebenen E₁ und E₂ durch das medizinische Instrument 12 verlaufen. Diese Ebenen E₁, E₂ werden jeweils aufgespannt durch die Längsachse x₃ des medizinischen Instruments 12 und die Längsachse x₁ bzw. x₂ eines der beiden Stäbe 131, 132, stehen im gezeigten Fall also senkrecht aufeinander. Während der Behandlung kann es jedoch sinnvoll sein, die Orientierung der dargestellten Schichtbilder zu verändern, um die das medizinische Instrument 12 umgebende Anatomie aus einer anderen Perspektive zu sehen. Um dies zu erreichen, muß der Arzt nur einfach an den als Drehkreuz ausgestalteten Einstellmitteln 13 drehen. Dadurch erreichen auch die an den Stäben 131 und 132 angebrachten LEDs 17 eine andere Position, was mittels des Positionsmeßsystems detektiert wird. Diese Information wird unmittelbar an die Steuereinheit 15 und die Recheneinheit 19 weitergeleitet, wo unmittelbar neue Schichtbilder erstellt und auf dem Monitor 16 dargestellt werden, die nun in den Ebenen E₃ und E₄ liegen (siehe Fig. 2b), welche durch die Achsen x₂ und x₃ bzw. x₁ und x₃ aufgespannt werden.

Es ist auch denkbar, die Einstellmittel 13 verkippbar auszugestalten, d.h. so auszugestalten, dass der Winkel zwischen der Längsachse x₃ des Instruments 12 und der durch die Stäbe 131 und 132 aufgespannten Ebene veränderbar ist. Dargestellt werden könnte dann beispielsweise immer die Schicht, die durch die Spitze des Instruments 12 verläuft und parallel zur durch die Stäbe 131 und 132 aufgespannten Ebene liegt.

Es kann vorgesehen sein, dass mit der gezeigten Röntgeneinrichtung auch während einer Behandlung Röntgenprojektionsbilder erstellt werden, die separat dargestellt oder mit dem präoperativ erfaßten Bilddatensatz kombiniert werden. Weiter kann vorgesehen sein, dass nach einer Veränderung der Einstellung der Einstellmittel 13 auch die Position des C-Bogens 1 verändert wird und ein neues Röntgenprojektionsbild aus einer anderen Perspektive erstellt wird.

In den Figuren sind lediglich beispielhafte Ausgestaltungen eines erfindungsgemäßen medizinischen Instruments als auch einer erfindungsgemäßen Anordnung gezeigt. Insbesondere für die Ausgestaltung der Einstellmittel bietet sich eine Vielzahl von Möglichkeiten an. Bei der in Fig. 1 gezeigten Anordnung kann das Positionsmeßsystem mit der Kameraeinheit und den Leuchtdioden vollständig entfallen, wenn der Teil des medizinischen Instruments mit den Einstellmitteln in regelmäßigen Zeitabständen während der Behandlung in Röntgenprojektionsaufnahmen abgebildet wird und daraus sich die aktuelle Einstellung der Einstellmittel bestimmen läßt. Eine andere Ausführungsform der erfindungsgemäßen Anordnung ist derart ausgestaltet, dass während der Behandlung keine Röntgeneinrichtung (oder eine andere bildgebende Einrichtung) vorgesehen ist, sondern lediglich ein Positionsmeßsystem zur Bestimmung der aktuellen Position des medizinischen Instruments und der aktuellen Einstellung der Einstellmittel sowie eine Bildverarbeitungseinheit mit einem Bildspeicher, auf dem ein präoperativ erfaßter Bilddatensatz gespeichert ist.

## Patentansprüche

1. Anordnung zur Darstellung von Schichtbildern während der Behandlung eines Untersuchungsobjekts (5) mit Meßmitteln (10, 14) zur Bestimmung der Position eines während der Behandlung in das Untersuchungsobjekt (5) teilweise eingeführten medizinischen Instruments (12) relativ zu einem Bezugssystem,
**dadurch gekennzeichnet, dass** an dem aus dem Untersuchungsobjekt (5) herausragenden Teil des medizinischen Instruments (12) Einstellmittel (13) angeordnet sind zur Veränderung der Orientierung der dargestellten Schichtbilder.

2. Anordnung nach Anspruch 1,
**dadurch gekennzeichnet, dass** die Einstellmittel (13) mit einer die Darstellung der Schichtbilder steuernden Steuereinheit (15) gekoppelt sind.

3. Anordnung nach Anspruch 1,
**dadurch gekennzeichnet, dass** die Einstellmittel (13) derart ausgestaltet sind, dass deren Einstellung mittels der Meßmittel (10) bestimmbar ist.

4. Anordnung nach Anspruch 1,
**dadurch gekennzeichnet, dass** die Einstellmittel (13) derart ausgestaltet sind, dass deren Einstellung auf einfache Weise manuell veränderbar ist.

5. Anordnung nach Anspruch 1,
**dadurch gekennzeichnet, dass** die Einstellmittel (13) eine Dreh- oder Schiebevorrichtung aufweisen, deren Einstellung die Orientierung der dargestellten Schichtbilder anzeigt und bestimmt.

6. Anordnung nach Anspruch 1,
**dadurch gekennzeichnet, dass** die Einstellmittel (13) einen im Griffbereich des medizinischen Instruments (12) angeordneten Drehschalter aufweisen.

7. Anordnung nach Anspruch 6,
**dadurch gekennzeichnet, dass** der Drehschalter (13) zwei kreuzförmig und senkrecht zur Längsachse (x₃) des medizinischen Instruments (12) angeordnete Stifte (131, 132) aufweist, wobei die Stifte (131, 132) um die Längsachse (x₃) des medizinischen Instruments (12) drehbar sind und die Orientierung zweier dargestellter Schichtbilder anzeigen, welche jeweils in einer durch die Längsachse (x₃) des medizinischen Instruments (12) und eine durch einen Stift (131, 132) verlaufende Stiftachse (x₁, x₂) aufgespannten Ebene liegen.

8. Anordnung nach Anspruch 7,
**dadurch gekennzeichnet, dass** der Winkel (α) zwischen den beiden Stiften (131, 132) veränderbar ist.

9. Anordnung nach Anspruch 7,
**dadurch gekennzeichnet, dass** der Winkel (α) zwischen den beiden Stiften (131, 132) 90° beträgt.

10. Anordnung nach Anspruch 1,
**dadurch gekennzeichnet, dass** die Orientierung der dargestellten Schichtbilder durch die Längsachse (x₃) des medizinischen Instruments (12) und die Einstellung der Einstellmittel (13) bestimmt wird.

11. Anordnung nach Anspruch 1,
**dadurch gekennzeichnet, dass** die Meßmittel (10, 14) ein Positionsmeßsystem, insbesondere ein optisches Positionsmeßsystem zur Bestimmung der räumlichen Positionen des medizinischen Instruments (12) und der Einstellmittel (13) aufweisen.

12. Anordnung nach Anspruch 1,
**dadurch gekennzeichnet, dass** die Schichtbilder aus einem vor der Behandlung erstellten dreidimensionalen Bilddatensatz, insbesondere einem Röntgenbilddatensatz, erstellt werden.

13. Anordnung nach Anspruch 1,
**dadurch gekennzeichnet, dass** eine Röntgeneinrichtung (1, 2, 3) vorgesehen ist zur Erstellung von Röntgenbildern während der Behandlung und dass mittels der Einstellmittel (13) die Orientierung der Röntgenbilder und der Schichtbilder veränderbar ist.

14. Anordnung nach Anspruch 13,
**dadurch gekennzeichnet, dass** die Einstellmittel (13) in den Röntgenbildern abbildbar ausgestaltet sind und dass die Meßmittel derart ausgestaltet sind, dass aus den Abbildungen der Einstellmittel (13) deren Einstellung bestimmbar ist.

15. Medizinisches Instrument (12) zur teilweisen Einführung in ein Untersuchungsobjekt (5) während einer Behandlung, wobei während der Behandlung Schichtbilder des Untersuchungsobjekts dargestellt werden sollen,
**dadurch gekennzeichnet, dass** an dem aus dem Untersuchungsobjekt (5) herausragenden Teil des medizinischen Instruments (12) Einstellmittel (13) angeordnet sind zur Veränderung der Orientierung der dargestellten Schichtbilder.

## Claims

1. An arrangement for displaying tomography images in the treatment of an object (5) under investigation with measuring means (10, 14) for determining the position relative to a reference system of a medical instrument (12) that has been partly inserted into the object (5) under investigation during said treatment,
**characterized in that** adjustment means (13) for changing the orientation of the displayed tomography images are arranged at the portion of the medical instrument (12) that projects from the object (5) under investigation.

2. An arrangement as claimed in claim 1,
**characterized in that** the adjustment means (13) are coupled to a control unit (15) that controls the display of the tomography images.

3. An arrangement as claimed in claim 1,
**characterized in that** the adjustment means (13) are arranged such that the adjustment thereof can be determined by the measuring means (10).

4. An arrangement as claimed in claim 1,
**characterized in that** the adjustment means (13) are arranged such that the adjustment thereof can be manually changed in a simple manner.

5. An arrangement as claimed in claim 1,
**characterized in that** the adjustment means (13) comprise a rotary or sliding arrangement whose adjustment signals and determines the orientation of the displayed tomography images.

6. An arrangement as claimed in claim 1,
**characterized in that** the adjustment means (13) comprise a rotary switch that is located within hand reach distance of the medical instrument (12).

7. An arrangement as claimed in claim 6,
**characterized in that** said rotary switch (13) comprises two rods (131, 132) arranged mutually crosswise and perpendicular to the longitudinal axis (x₃) of the medical instrument (12), which rods (131, 132) are rotatable about the longitudinal axis (x₃) of the medical instrument (12) and signal the orientation of two displayed tomography images, which images each lie in a plane defined by the longitudinal axis (x₃) of the medical instrument (12) and a rod axis (x₁, x₂) extending through the respective rod (131, 132).

8. An arrangement as claimed in claim 7,
**characterized in that** the angle (α) enclosed by the two rods (131, 132) can be changed.

9. An arrangement as claimed in claim 7,
**characterized in that** the angle (α) enclosed by the two rods (131, 132) is 90°.

10. An arrangement as claimed in claim 1,
**characterized in that** the orientation of the displayed tomography images is defined by the longitudinal axis (x₃) of the medical instrument (12) and the setting of the adjustment means (13).

11. An arrangement as claimed in claim 1,
**characterized in that** the measuring means (10, 14) comprise a position measuring system, in particular an optical position measuring system, for determining the spatial positions of the medical instrument (12) and the adjustment means (13).

12. An arrangement as claimed in claim 1,
**characterized in that** the tomography images are reconstructed from a three-dimensional set of image data obtained before the treatment, in particular from a set of X-ray image data.

13. An arrangement as claimed in claim 1,
**characterized in that** an X-ray apparatus (1, 2, 3) is provided for generating X-ray images during said treatment, and **in that** the orientation of the X-ray images and the tomography images can be changed through the adjustment means (13).

14. An arrangement as claimed in claim 13,
**characterized in that** the adjustment means (13) are arranged such that they can be imaged in the X-ray images, and **in that** the measuring means are arranged such that the setting of the adjustment means (13) can be derived from the images thereof.

15. A medical instrument (12) to be partly inserted into an object (5) under investigation for the purpose of displaying tomography images of said object under investigation during the treatment,
**characterized in that** adjustment means (13) for changing the orientation of the displayed tomography images are arranged at the portion of the medical instrument (12) that projects from the object (5) under investigation.

## Revendications

1. Dispositif pour représenter des images tomographiques pendant le traitement d'un objet d'analyse (5) avec des moyens de mesure (10, 14) pour déterminer la position d'un instrument médical (12) inséré partiellement pendant le traitement dans l'objet d'analyse (5) par rapport à un système de référence, **caractérisé en ce que,** sur la partie de l'instrument médical (12) dépassant de l'objet d'analyse (5) se trouvent des moyens de réglage (13) pour modifier l'orientation des images tomographiques représentées.

2. Dispositif selon la revendication 1, **caractérisé en ce que** les moyens de réglage (13) sont couplés à une unité de commande (15) commandant la représentation des images tomographiques.

3. Dispositif selon la revendication 1, **caractérisé en ce que** les moyens de réglage (13) sont conçus de telle sorte que leur réglage peut être déterminé à l'aide des moyens de mesure (10).

4. Dispositif selon la revendication 1, **caractérisé en ce que** les moyens de réglage (13) sont conçus de telle sorte que leur réglage peut être modifié manuellement de façon simple.

5. Dispositif selon la revendication 1, **caractérisé en ce que** les moyens de réglage (13) présentent un dispositif de rotation ou de glissement dont le réglage affiche et détermine l'orientation des images tomographiques représentée.

6. Dispositif selon la revendication 1, **caractérisé en ce que** les moyens de réglage (13) présentent un commutateur rotatif disposé dans la zone de préhension de l'instrument médical (12).

7. Dispositif selon la revendication 6, **caractérisé en ce que** le commutateur rotatif (13) présente deux tiges (131, 132) disposées en forme de croix et perpendiculairement à l'axe longitudinal (x₃) de l'instrument médical, les tiges (131, 132) pouvant tourner autour de l'axe longitudinal (x₃) de l'instrument médical (12) et affichant l'orientation de deux images tomographiques représentées qui se trouvent respectivement dans un plan couvrant l'axe longitudinal (x₃) de l'instrument médical (12) et un axe de tige (x₁, x₂) passant par une tige (131, 132).

8. Dispositif selon la revendication 7, **caractérisé en ce que** l'angle (α) entre les deux tiges (131, 132) peut être modifié.

9. Dispositif selon la revendication 7, **caractérisé en ce que** l'angle (α) entre les deux tiges (131, 132) est de 90°.

10. Dispositif selon la revendication 1, **caractérisé en ce que** l'orientation des images tomographiques représentées est déterminée par l'axe longitudinal (x₃) de l'instrument médical (12) et le réglage des moyens de réglage (13).

11. Dispositif selon la revendication 1, **caractérisé en ce que** les moyens de mesure (10, 14) présentent un système de mesure de position, en particulier, un système de mesure de position optique pour déterminer les positions spatiales de l'instrument médical (12) et des moyens de réglage (13).

12. Dispositif selon la revendication 1, **caractérisé en ce que** les images tomographiques sont produites à partir d'un jeu d'images tomographiques tridimensionnelles générées avant le traitement, en particulier, un jeu d'images radiographiques.

13. Dispositif selon la revendication 1, **caractérisé en ce qu'**un dispositif radiographique (1, 2, 3) est prévu pour générer des images radiographiques pendant le traitement et que, à l'aide des moyens de réglage (13), l'orientation des images radiographiques et des images tomographiques peut être modifiée.

14. Dispositif selon la revendication 13, **caractérisé en ce que** les moyens de réglage (13) sont conçus de façon à pouvoir être représentés dans les images radiographiques et que les moyens de mesure sont conçus de telle sorte que leur réglage puisse être déterminé à partir des représentations des moyens de réglages (13).

15. Instrument médical (12) pour l'introduction partielle dans un objet d'analyse (5) pendant un traitement, où pendant le traitement, des images tomographiques de l'objet d'analyse doivent être représentées, **caractérisé en ce que** sur la partie de l'instrument médical (12) dépassant de l'objet d'analyse (5), des moyens de réglage (13) sont placés pour modifier l'orientation des images tomographiques représentées.
